# EUROPEAN PATENT APPLICATION

(11) **EP 0 705 895 A1**
(43) Date of publication of application: **10.04.1996**
(21) Application number: 95115567.0
(22) Date of filing: 02.10.1995
(51) Int. Cl.: C09J 103/06, A61L 15/58, D04H 1/64

(54) **Starch based hot melt adhesives for nonwoven applications**

(30) Priority: 05.10.1994 US 317682
(71) Applicant: National Starch and Chemical Investment Holding Corporation, Wilmington, Delaware 19809 (US)
(72) Inventor: Riswick, Martin, Marlow (GB)
(74) Representative: Hagemann, Heinrich, Dr. rer. nat., Dipl.-Chem.

(57) **Abstract**

A process for bonding tissue or nonwoven to similar or dissimilar substrates comprising the step of applying to the substrate a molten hot melt adhesive composition, said adhesive comprising 20 to 60% by weight of an intermediate or high DS starch ester having from about 2 to 18 carbon atoms in the ester component and a DS (degree of substitution) of from about 0.3 to 2.5, 5 to 40% by weight of a polar wax, 5 to 50% by weight of a plasticizing diluent, 0 to 25% by weight of a compatible tackifier and 0 to 3% by weight of an antioxidant; and bonding another substrate thereto.

## Description

A nonwoven fabric is defined as an interlocking fiber network characterized by flexibility, porosity and integrity. The individual fibers used to compose the nonwoven fabric may be synthetic, naturally occurring, or a combination of the two. The individual fibers may be mechanically, chemically, or thermally bonded to each other. Nonwovens are used commercially for a variety of applications including insulation, packaging, household wipes, surgical drapes, medical dressings, and in disposable articles such as diapers, adult incontinent products and sanitary napkins. Tissue is a closely related material in which the individual fibers may or may not be chemically bonded to one another.

In many of the aforementioned applications it is necessary to adhere the nonwoven or tissue to another substrate or component. The second substrate may be another nonwoven, tissue, or an unrelated material. A commonly employed technique to bond the assembly together is the use of a hot melt adhesive. Hot melt adhesives allow for cost and time efficient manufacturing since there is no evaporation step necessary as is the case for water based or solvent based adhesive systems. Suitable hot melt adhesives must possess excellent adhesion to the substrates involved. For nonwoven applications they must also possess good flexibility (or hand), no staining or bleed through, suitable viscosity, set speed and open time to function on commercially available equipment and finally, acceptable thermal aging properties.

Recently a variety of nonwoven and tissue applications have been developed which require that the hot melt adhesive demonstrate appreciable water solubility, dispersibility or sensitivity. In these situations the hot melt adhesive must provide a durable bond to the nonwoven or tissue until exposed to a predetermined condition (e.g., room temperature water), after which the adhesive would release from the substrate(s). This ambient water releasability is a particularly desirable property in the disposable market where flushability and/or degradeability are becoming critical.

Such a water soluble, dispersible or sensitive composition would also find utility as a binder to chemically bond the nonwoven fibers together in the manufacture of the nonwoven fabric or tissue. Such a thermoplastic, water sensitive binder would allow for high speed manufacturing and would produce a nonwoven or tissue which would disintegrate under a desired condition.

It has now been discovered that a specific class of hot melt adhesives and/or binders can be prepared from 20 to 60% by weight of an intermediate or high DS starch ester having from 2 to 18 carbon atoms in the ester component and a DS (degree of substitution) of from 0.3 to 2.5, 5 to 40% by weight of a polar wax, 5 to 50% by weight of a plasticizing diluent, 0 to 25% by weight of a tackifier and 0 to 3% by weight of an antioxidant.

Adhesives of this type are disclosed in copending application Serial No. 07/995,493, filed December 23, 1992.

These adhesives, while sufficiently polar in nature to be water sensitive and release when exposed to water provide, nonetheless sufficient fast-setting as well as acceptable flexibility and machining properties to be used in commercial disposable applications. In particular, their ability to be readily dispersed in water greatly facilitates clean-up of dried spills on the machines and surrounding work areas. Previously, it was necessary to use solvent for such clean-up applications.

These starch based hot melt adhesives are particularly useful in constructing relatively thin disposable nonwovens such as diapers and sanitary napkins, the adhesive being employed to adhere the nonwoven and/or polyethylene substrates to bond multiple layers of the thin nonwoven absorbent substrates, and also to incorporate therein varying amounts of super-absorbent materials. These super-absorbent materials are often used in fine particulate form, it being necessary that these particles be permanently incorporated into the final construction. In such cases, the hydrophilic nature of the starch used adhesive facilitates the transmission of the fluid into the absorbent layers. In contrast, most hot melt adhesives are hydrophobic, a characteristic which serves to hinder the flow of fluid through the construction.

The main or primary constituent of the hot melt adhesive composition used herein is a modified starch ester. The modified starch which can be used is an esterified starch compound having the formula: 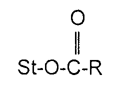
where St represents the starch base material and R is an alkyl, aryl, alkenyl, alkaryl or aralkyl of 1 to 17, preferably 1 to 6 carbon atoms. More preferably, the ester compound will contain an R group which is an alkyl of 1 to 2 carbon atoms. Starch esters of this type include starch acetate, starch propionate, starch butyrate, starch hexanoate, starch stearate, starch oleate, starch benzoate, blends of two more of these esters, for example starch acetate/starch propionate and mixed starch esters where the starch contains two or more different ester substituents, e.g., starch acetate/propionate, i.e., the ester having the formula such as: 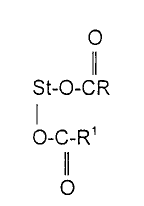
where R and R¹ represent different substituent groups as defined above.

Additionally, the modified starch ester will have a DS (degree of substitution) of from 0.3 to 3.0, preferably from 0.7 to 2.4, and more preferably from 0.8 to 2.0. The term "degree of substitution" (DS) as used herein indicates the average number of sites per anhydroglucose unit of the starch molecule on which there are substituent groups.

The starch esters are prepared from the respective carboxylic acid anhydride or acid chlorides. Typical methods include reactions in aqueous systems as disclosed in U.S. Patent No. 2,461,139 issued February 8, 1949 to C. Caldwell and in solvent systems such as pyridine. These and other methods are disclosed in "Modified Starches: Properties and Uses", edited by O. B. Wurzburg, Chapter 4, pp. 55-77, 1986 and "Starch: Chemistry and Technology", edited by R. L. Whistler, et al., Chapter X, pp. 332-343, 1984 as well as in copending application Serial No. 07/995,493, filed December 23, 1992.

The base starch material used in the modified starch esters may be any of several starches, native, converted or derivatized. Such starches include those derived from any plant source including corn, potato, wheat, rice, sago, tapioca, waxy maize, sorghum and high amylose starch such as high amylose corn, i.e., starch having at least 45% and more particularly at least 65% amylose content by weight, etc. Starch flours may also be used. Also included are the conversion products derived from any of the former bases, such as, for example, dextrins prepared by hydrolytic action of acid and/or heat; fluidity or thin boiling starches prepared by enzyme conversions or mild acid hydrolysis; oxidized starches prepared by treatment with oxidants such as sodium hypochlorite; and derivatized starches, such as, cationic, anionic, amphoteric, non-ionic, and crosslinked. While any of these starches may be used, the high amylose starches and particularly those having amylose content of at least 65% by weight are preferred. Although the full molecular weight or unhydrolyzed starches can be used as the base material, particularly useful are those starches which have been hydrolyzed but not severely degraded. Such starches have a dextrose equivalent (DE) of less than about 10 and preferably less than about 5. Dextrose equivalent (DE) is defined as the reducing power of the hydrolyzate. Each starch molecule has one reducing end, therefore DE is inversely related to molecular weight. The DE of anhydrous D-glucose is defined as 100 and the DE of unhydrolyzed starch is virtually zero.

In addition to the ester component, a plasticizing diluent is present in the hot melt adhesive formulation. Suitable as plasticizing diluents are non-volatile organic materials which are compatible with the modified starch ester and will be present in sufficient amount to allow the formulation to function as a hot melt by melting and forming a homogeneous melt at the application temperature and having a suitable viscosity at that temperature. This means the use of diluent will allow the formulation to melt at the application temperature, i.e., 400°F (204°C) or less, and also possess the desired viscosity of <50,000 cP at that temperature. More particularly, the plasticizing diluent will be an organic material which is non-volatile and compatible with the starch ester and is characterized in containing one or more polar groups, i.e.,it is not an all hydrocarbon material. Typically it will have a molecular weight of 5,000 or less. Useful plasticizing diluents containing polar groups include sulfonamides, carboxylic acids and esters, carboxylate salts, amides, phosphate esters, alcohols, i.e., hydroxy containing compounds, epoxides, sulfones, ethers, imides, amines, carbonates, ureas and urethanes. The preferred diluents include those containing sulfonamide, alcohol, amide and ester groups which absorb low levels of moisture at high humidity, i.e., have a moisture content of less than about 20%, preferably less than about 15% by weight, at 90% relative humidity (RH) and 23°C. Particularly preferred diluents are the alcohols or hydroxy containing compounds having this characteristic of low moisture absorption, i.e., hydrophobic alcohols and especially the ethoxylates of phenol and bisphenol A, and N-(2-hydroxyethyl)-12-hydroxy stearamide. The preferred diluents do not include the hydrophilic type alcohols such as glycerin or sorbitol and other compounds of this type which are hygroscopic and easily pick up and absorb moisture. The plasticizing diluent is generally present in amounts of 5 to 50%, preferably 20 to 40% by weight, of the hot melt adhesive.

The adhesive also contains 5 to 40%, preferably 10 to 30% by weight, of a compatible polar wax. Particularly useful are diluents selected from the group consisting of glycerol mono- and distearate, synthetic long chain linear polymeric alcohols, stearic acid, high acid number waxlike materials of mono- or dicarboxylic acids, fatty amide such as the mono-ethanol amide of hydroxy stearic acid, acid wax derived from montan wax, stearyl alcohol, hydrogenated castor oil, ethoxylated alcohols, 12(OH) stearic acid and stearic-cetyl alcohol.

The adhesive compositions may also include tackifier resins which, if present are used in amounts of 5 to 25% by weight, based on the weight of the composition. The tackifying resins useful in the adhesive compositions are generally polar in nature and have a Ring and Ball softening point (as described by ASTM E-26) of greater than 60°C and include rosin and rosin derivatives, terpene phenolics, pure phenolic resins, and the like. More particularly, the useful tackifying resins include any compatible resins or mixtures thereof such as (1) natural and modified rosins such, for example, as gum rosin, wood rosin, tall oil rosin, distilled rosin, hydrogenated rosin, dimerized rosin, and polymerized rosin; (2) glycerol and pentaerythritol esters of natural and modified rosins, such, for example, as the glycerol ester of pale, wood rosin, the glycerol ester of hydrogenated rosin, the glycerol ester of polymerized rosin, the pentaerythritol ester of hydrogenated rosin, and the phenolic-modified pentaerythritol ester of rosin; (3) phenolic modified terpene resins and hydrogenated derivatives thereof such, for example, as the resin product resulting from the condensation, in an acidic medium, of a bicyclic terpene and a phenol; (4) thermoplastic alkyl phenolic resins such as those described in U.S. Patent Nos. 4,073,776 and 4,023,826. Mixtures of two or more of the above described tackifying resins, as well as blends of the above resins with small amounts of (e.g., less than about 10% of the adhesive) less compatible resins may be utilized for some formulations.

Optionally, the adhesive composition may also contain up to about 35% of a compatible hydrophilic or hydrophobic thermoplastic polymer. Suitable polymers include hydrophilic polymers such as water-soluble and/or water-swellable polymers and hydrophobic thermoplastic water-insoluble polymers. Such polymers include celluloses such as alkylcelluloses, hydroxyalkyl-celluloses, cellulose esters and cellulose salts, polyvinyl alcohols prepared by partial to essentially complete hydrolysis of polyvinyl acetate (preferably 45 to 80% hydrolyzed), synthetic polymers such as poly(acrylic acids) and their salts and esters, poly(acrylamides), poly(vinyl acetates), poly(vinyl acetate phthlates), poly(vinyl pyrrolidone), poly(crotonic acids), vinylpolymers such as polyvinylacetates, polyacrylonitriles, polyvinylcarbazoles, polyacetals, polycondensates such as polyamides, thermoplastic polyesters such as polyhydroxybutyrate/hydroxy-valerate, polylactides (i.e., esters of lactic acid), polyurethanes, poly(alkylene terephthalates), polyarylethers, poly(ethyl oxazoline), poly(ethylene imine), poly(ethylene glycol), thermoplastic polyimides, poly(alkylene oxides) such as polymers of ethylene oxide and propylene oxide, and gelatin.

Particularly useful are polymers containing polar groups such as those described earlier for the diluent with those containing hydroxyl groups being most preferred, especially polyvinyl alcohol, ethylene/vinyl alcohol and hydroxypropyl cellulose.

An antioxidant or stabilizer may also be included in the adhesive compositions described herein in amounts of up to about 3% by weight. Among the applicable antioxidants or stabilizers are high molecular weight hindered phenols and multifunctional phenols such as sulfur and phosphorous-containing phenols. Representative hindered phenols include: 1,3,5-trimethyl 2,4,6-tris (3,5-di-tert-butyl-4-hydroxy-benzyl)benzene; pentaerythritoltetrakis-3(3,5-di-tert-butyl-4-hydroxyphenyl)-propionate; n-octadecyl-3,5-di-tert-butyl-4-hydroxyphenol)-propionate; 4,4'-methylenebis (2,6-tert-butylphenol); 4,4'-thiobis (6-tert-butyl-o-cresol); 2,6-di-tertbutylphenol; 6-(4-hydroxyphenoxy)-2,4-bis(n-octyl-thio)-1,3,5-triazine; di-n-octadecyl 3,5-di-tert-butyl-4-hydroxy-benzyl-phosphonate; 2-(n-octylthio)-ethyl 3,5-di-tert-butyl-4-hydroxy-benzoate; and sorbitol hexa[3-(3,5-di-tert-butyl-4-hydroxyphenyl)-propionate].

Other additives conventionally used in hot melt adhesives to satisfy different properties and meet specific application requirements also may be added to the adhesive composition of this invention. Such additives include fillers, pigments, flow modifiers, dyestuffs, etc., which may be incorporated in minor or larger amounts into the adhesive formulation, depending on the purpose.

These hot melt adhesives may be prepared using techniques known in the art. Typically, the adhesive compositions are prepared by blending the components in the melt at a temperature of 100 to 200°C until a homogeneous blend is obtained, approximately two hours. Various methods of blending are known and any method that produces a homogeneous blend is satisfactory. In blending the starch with other components of the hot melt composition, it may be desirable to precondition or preswell the starch with the plasticizing diluent (a portion, i.e., 10% or more, or all of the diluent used in the formulation by weight) or water, preferably with both, either at room temperature or slightly elevated temperature (typically ≦ 100°C) and preferably in a sealed container. The added moisture (usually ≦ 35% by weight based on the weight of the starch) promotes swelling of the starch and thus serves as a processing aid. Moisture can be added directly to the starch as liquid water or by conditioning the starch at high humidity. Essentially all of the water is removed by evaporation (to < 2% by weight of the final composition) in the final mixing of the adhesive formulation. Preswelling of the starch promotes the rapid formation of a homogeneous melt at reduced temperatures and has been found particularly useful when the starch has a low degree of substitution, such as a DS of less than 2.

The resulting adhesives are characterized in that they have a viscosity of 50,000 cP or less at the application temperature of 400°F (204°C) or less. The viscosity as used herein is a Brookfield viscosity measured using a Brookfield viscometer model No. DV-II with spindle no. 27 at 10 rpm.

The resulting adhesives of the present invention are characterized by their ability to provide a durable bond to a nonwoven or tissue article and otherwise meet the unique requirements of the application (including flexibility, non- staining, and machinable viscosity) and later release upon exposure to water at ambient temperatures after a desired residence period. The adhesives described herein also possess exceptional thermal stability which distinguishes them from other moisture sensitive technologies. Further, their hydrophilic natures facilitated ready transmission of the fluid throughout the construction. Additionally, many of the compositions described herein display long open time and pressure sensitivity necessary for spray or melt blown application methods.

The adhesive product can be applied to a substrate such as a nonwoven article or tissue by a variety of methods including coating or spraying in an amount sufficient to cause the article to adhere to another substrate such as tissue, nonwoven, or an unrelated material such as a low density polyolefin or other conventionally employed water impermeable substrate. When exposed to water of a specific temperature for a specific period of time, the nonwoven or tissue releases.

The following examples illustrate the production of suitable hot melt adhesives or binders as well as the use thereof in a variety of applications. In the examples, all parts are by weight and all temperatures in degree Celsius unless otherwise noted. Test procedures used herein are as follows:

### Test Procedures

Melt viscosities of the hot melt adhesives/binders were determined on a Brookfield RVT Thermosel viscometer using a number 27 spindle at 20 or 50 rpms.

The heat stability of the adhesive/binder samples was determined in the following manner: a 60 gram sample was stored in a covered jar at 175°C for 72 hours and observed for the formation of gel, skin or any deleterious change in color. The viscosity change upon this thermal aging was then determined as stated above.

Polypropylene nonwoven to nonwoven bonds used in testing nonwoven adhesion and water sensitivity were prepared by applying a 1.0 to 1.5 mm wide (compressed) bead of molten hot melt at 350 to 375°F to one piece of 2" x 6" nonwoven and mating it to a second piece of nonwoven of the same size. Open time was <2 seconds and dwell time 10 seconds. These bonds were then aged at 70°F/50% RH. Bonds were also tested similarly for tissue to nonwoven and tissue to tissue applications.

Nonwoven adhesion of the various samples was determined by pulling polypropylene nonwoven to polypropylene nonwoven bonds apart in a peel mode at 12"/min on an Instron tester. The bond is characterized by the presence or absence of fiber tear (F.T.) and/or the amount of resistance (lbs/in). If F.T. was noted on all three samples, the amount of resistance was not recorded. Conventional hot melt adhesives used for bonding nonwovens typically display fiber tearing bonds (F.T.) and/or resistance ≧0.2 lb/in.

### 180° T Peel Testing Procedure

The samples are prepared as follows. A glue line was extruded onto nonwoven at approximately 300 to 325°F with a line speed of 100 FPM to form a glue line approximately 0.5 mm wide with a coating weight of approximately 2 to 4 mg/linear inch. A second nonwoven substrate was immediately bonded to the glue bead with bonding pressure of approximately 60 psi. Samples were then cut parallel to adhesive lines, leaving at least 1/8" on each side of the exterior adhesive lines. The samples were conditioned overnight at 70°F/50% RH constant temperature and humidity. Testing was also done on tissue to tissue samples.

Instron Testing: The ends of each sample were taped, then placed in jaws, with the adhesive coated nonwoven in the stationary jaw. The sample was pulled at 12 in/min crosshead speed, 2 in/min chart speed in 180° T peel mode and the average peel value recorded in grams or pounds for each product tested. If there was bond failure, the type of failure was recorded instead of peel value.

### EXAMPLE I

A useful starch based hot melt adhesive was prepared by the following method.

Starch acetate ester having a degree of substitution of 1.5 (Superfluidity Hylon VII from National Starch and Chemical Company), ethyloxylated Bisphenol A (Macol 206EM from PPG), and the antioxidants were added to a (130°C) preheated sigma blade mixer and blended until smooth. The wax component (Paracin 220 from CasChem) was then added and mixed until fully incorporated.

**TABLE I**

| | |
|---|---|
| Starch Acetate | 43 |
| Macol 206EM | 49 |
| Paracin 220 | 10 |
| Irgafos 169 (Ciba Additives) | 0.5 |

The resulting adhesive was then tested for the desired performance properties using the procedures described previously. The results are shown in Table II.

**TABLE II**

| **TEST RESULTS** | **250°F** | **275°F** |
|---|---|---|
| VISCOSITY | 3200 cps | 2100 cps |

| **HEAT STABILITY** | **275°F (24 hr)** | **275°F (72 hr)** |
|---|---|---|
| Skin | None | None |
| Edge Ring | None | None |
| Char | None | None |
| Color | Light Tan | Brown |
| Gel | None | None |
| Viscosity | 2170 | 2050 |
| OV (%) | +3.58 | -2.16 |

| **INSTRON** | **Substrate Failure** | **Average Peel** |
|---|---|---|
| Tissue/Tissue | All tissue failure | -- |
| | No bond failure | |
| Tissue/Nonwoven | Tissue substrate failure | -- |
| | No bond failure | |
| Nonwoven/Nonwoven | | 108 g |
| | ---- | |

In addition to the testing described above, nonwoven substrates coated with 10 grams per square meter of the adhesive were subjected to standard testing to determine the degree of penetration/absorption of synthetic urine through the coated substrate when the substrate was placed on an inclined surface. The results indicate that nonwovens coated with the starch based adhesive exhibited virtually complete absorption of three doses (5 ml each) of the liquid as compared with the same nonwoven substrate which had not been coated with adhesive wherein 30% of the second dosage was not absorbed and 90% of the third dosage ran off the inclined surface.

These results clearly demonstrate the suitability of the starch based adhesives for nonwoven and other disposable applications. Similar results would be expected using starch based adhesives prepared from other starch esters or containing other compatible formulating materials.

In summary, the results show that these hot melt adhesives may be successfully used to form nonwoven disposable product as described hereinabove. It will be apparent that various changes and modifications may be made in the embodiments of the invention described above, without departing from the scope of the invention, as defined in the appended claims, and it is intended therefore, that all matter contained in the foregoing description shall be interpreted as illustrative only and not limitative of the invention.

## Claims

1. A process for bonding tissue or nonwoven to similar or dissimilar substrates comprising the step of applying to the substrate a molten hot melt adhesive composition, said adhesive comprising 20 to 60% by weight of an intermediate or high DS starch ester having from about 2 to 18 carbon atoms in the ester component and a DS (degree of substitution) of from about 0.3 to 2.5, 5 to 40% by weight of a polar wax, 5 to 50% by weight of a plasticizing diluent, 0 to 25% by weight of a compatible tackifier and 0 to 3% by weight of an antioxidant; and bonding another substrate thereto.

2. The process of Claim 1 wherein the starch ester in the adhesive has a DS of 0.7 to 2.4.

3. The process of Claim 2 wherein the starch ester in the adhesive contains 2 to 7 carbon atoms in the ester component.

4. The process of Claim 1 wherein the starch ester in the adhesive has the formula: 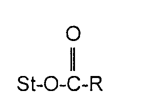 where St is the starch base material and R is an alkyl, aryl, alkenyl, alkaryl or aralkyl containing 1 to 17 carbon atoms.

5. The process of Claim 1 wherein the tackifying resin in the adhesive is selected from the group consisting of rosin, rosin derivatives, phenolic modified coumarone indene resins, coumarone indene resins with softening points of about 5 to 117°C and phenolic modified terpene resins.

6. The process of Claim 1 wherein the compatible polar wax in the adhesive is selected from the group consisting of glycerol mono- and distearate, synthetic long chain linear polymeric alcohols, stearic acid, high acid number waxlike materials of mono- or dicarboxylic acids, fatty amides, acid wax derived from montan wax, stearyl alcohol, hydrogenated castor oil, ethoxylated alcohols, 12(OH) stearic acid and stearic-cetyl alcohol.

7. The process of Claim 1 wherein the starch in the adhesive is an acetate ester of high amylose and the plasticizing diluent is an ethoxylate of bisphenol A.

8. The process of Claim 9 wherein the nonwoven substrate has incorporated therein superabsorbent particles.

9. The process of Claim 1 wherein one substrate is a nonwoven and the other substrate is a low density polyolefin.

10. A disposable product containing incorporated therein at least one nonwoven substrate containing superabsorbent particulate polymers, wherein the nonwoven substrate is bonded into the disposable product using a hot melt adhesive having a Tg of -40 to +40°C and comprising 20 to 60% by weight of an intermediate or high DS starch ester having from about 2 to 18 carbon atoms in the ester component and a DS (degree of substitution) of from about 0.3 to 2.5, 5 to 40% by weight of a polar wax, 5 to 50% by weight of a plasticizing diluent, 0 to 25% by weight of a compatible tackifier and 0 to 3% by weight of an antioxidant.
